(19) Europäisches Patentamt
European Patent Office
Office européen des brevets

(11) EP 3 117 821 B1

(12) # EUROPEAN PATENT SPECIFICATION

(45) Date of publication and mention of the grant of the patent:
**26.12.2018 Bulletin 2018/52**

(21) Application number: **14885814.5**

(22) Date of filing: **14.11.2014**

(51) Int Cl.:
***A61K 8/68*** (2006.01) ***A61K 8/06*** (2006.01)
***A61K 8/34*** (2006.01) ***A61K 8/37*** (2006.01)
***A61Q 19/00*** (2006.01) ***A61K 8/39*** (2006.01)

(86) International application number:
**PCT/JP2014/080157**

(87) International publication number:
**WO 2015/136778 (17.09.2015 Gazette 2015/37)**

(54) **METHOD FOR PRODUCING CERAMIDE DISPERSION COMPOSITION**

VERFAHREN ZUR HERSTELLUNG EINER CERAMIDDISPERSIONSZUSAMMENSETZUNG

PROCÉDÉ DE PRODUCTION DE COMPOSITION DE DISPERSION DE CÉRAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.03.2014 JP 2014052464**

(43) Date of publication of application:
**18.01.2017 Bulletin 2017/03**

(73) Proprietor: **Fujifilm Corporation**
**Minato-ku**
**Tokyo 106-8620 (JP)**

(72) Inventor: **KITAOKA, Hiroyuki**
**Ashigarakami-gun**
**Kanagawa 258-8577 (JP)**

(74) Representative: **Klunker IP**
**Patentanwälte PartG mbB**
**Destouchesstraße 68**
**80796 München (DE)**

(56) References cited:
**EP-A1- 2 361 678**
**EP-A1- 2 556 821**
**JP-A- 2001 316 217**
**JP-A- 2006 335 693**
**JP-A- 2008 120 731**
**US-A1- 2006 057 091**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 117 821 B1

## Description

## BACKGROUND OF THE INVENTION

1. Field of the Invention

The present invention relates to a method for manufacturing a ceramide dispersion composition.

2. Description of the Related Art

Ceramides are in the stratum corneum of skin and play an important role in retaining moisture by constructing a lipid barrier necessary for retaining moisture. There are different types of ceramide in the human skin, and they perform different functions. In recent years, in anticipation of the skin care effect of ceramides, various ceramide formulation techniques have been developed.

As a technique for stably formulating a ceramide at a high concentration, a technique is known in which a ceramide in a specific proportion, a surfactant having an HLB value of equal to or greater than 9, an oleaginous component staying in a liquid form at 25°C, and water are emulsified at a treatment pressure of equal to or higher than 50 MPa (for example, see JP2006-335693A).

Furthermore, JP2013-224314A discloses a method for manufacturing a ceramide dispersion composition which contains a ceramide at high concentration and has excellent dispersion stability through a process in which an oil phase is prepared by dissolving an oleaginous component containing a specific ceramide in a water-soluble organic solvent, and the obtained oil phase and a water phase are separately passed through micro-flow channels and then mixed together by collision under counterflow. Patent document US 2006/0057091 discloses the preparation of a semi-transparent cosmetic product that contains ceramides.

## SUMMARY OF THE INVENTION

In order for the skin care effect of ceramides to be fully exerted, the skin penetration of ceramides needs to be improved. Considering the skin penetration of ceramides, it is desired for ceramide dispersion particles to have a small particle size. Although ceramides can be dispersed using a surfactant or the like, it is difficult to sufficiently reduce the particle size thereof to a sufficient degree. For example, from the viewpoint of the skin penetration, it cannot be said that the ceramide dispersion particles contained in the emulsion composition disclosed in JP2006-335693A have a small particle size. According to the method disclosed in JP2013-224314A, it is possible to obtain ceramide dispersion particles having a fine particle size, but it cannot be said that it is a simple method because a special device is required and a desolventizing treatment needs to be performed after dispersion.

The present invention has been made in consideration of the circumstances described above, and an object thereof is to provide a ceramide dispersion composition, which contains fine ceramide dispersion particles and exhibits an excellent skin care effect when being used in an external preparation for skin such as cosmetics, by a simple method.

Specific means for achieving the aforementioned object is as follows.

<1> A method for manufacturing a ceramide dispersion composition, comprising obtaining a crude dispersion liquid by performing a dispersion treatment in a state where a mixture, which contains ceramide, a nonionic surfactant, and a polyhydric alcohol in an amount of 10 times to 12,000 times as much as the mass of the ceramide, is heated to a temperature of equal to or higher than 100°C, and performing a high-pressure emulsification treatment after mixing the crude dispersion liquid with water or a water-containing composition.

<2> The method for manufacturing a ceramide dispersion composition described in <1>, in which the polyhydric alcohol is at least one kind of polyhydric alcohol selected from the group consisting of glycerin and a diol compound.

<3> The method for manufacturing a ceramide dispersion composition described in <2>, in which the polyhydric alcohol is glycerin and at least one kind of diol compound.

<4> The method for manufacturing a ceramide dispersion composition described in any one of <1> to <3>, in which lecithin is used.

<5> The method for manufacturing a ceramide dispersion composition described in any one of <1> to <4>, in which the nonionic surfactant is a polyglycerin fatty acid ester.

<6> The method for manufacturing a ceramide dispersion composition described in any one of <1> to <5>, in which the ceramide dispersion composition does not contain an oleaginous component staying in a liquid form at 25°C or contains an oleaginous component staying in a liquid form at 25°C in an amount of equal to or less than 1% by mass.

<7> The method for manufacturing a ceramide dispersion composition described in any one of <1> to <6>, in which the ceramide is a compound represented by the following Formula (I).

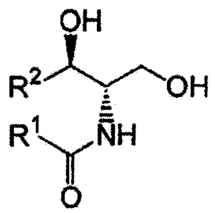

... (I)

In Formula (I), $R^1$ represents a linear or branched hydrocarbon group which has 40 to 55 carbon atoms in total, contains an ester bond in a carbon chain, and may contain one partial structure or two or more partial structures selected from the group consisting of a multiple bond, an aromatic ring, and an aromatic heterocyclic ring in the carbon chain; and $R^2$ represents an alkyl group having 11 to 21 carbon atoms that may have a hydroxyl group or an alkenyl group having 11 to 21 carbon atoms that may have a hydroxyl group.

In the present specification, a range of numerical values described using "to" means a range including the numerical values listed before and after "to" as an upper limit and a lower limit respectively.

In the present specification, in a case where there is a plurality of substances corresponding to each component in a composition, unless otherwise specified, the amount of each component in the composition means the total amount of the plurality of substances present in the composition.

In the present specification, the term "step" includes not only an independent step but also a step which cannot be clearly differentiated from other steps as long as the intended purpose thereof is achieved.

In the present specification, "ceramide dispersion particles" mean ceramide-containing particles which contain at least a ceramide as a constituent and are dispersed as a disperse phase in a continuous phase.

In the present specification, an expression such as "(poly)glycerin fatty acid ester" includes all of a glycerin fatty acid ester which contains one glycerin unit and one fatty acid unit, a glycerin fatty acid ester which contains either a plurality of glycerin units or a plurality of fatty acid units, and a glycerin fatty acid ester which contains a plurality of glycerin units and a plurality of fatty acid units in combination, and is used in a case where these glycerin fatty acid esters are used without distinction.

According to the present invention, it is possible to provide a ceramide dispersion composition, which contains fine ceramide dispersion particles and exhibits an excellent skin care effect when being used as an external preparation for skin such as cosmetics, by a simple method.

**DESCRIPTION OF THE PREFERRED EMBODIMENTS**

Hereinafter, specific embodiments of the present invention will be described in detail, but the present invention is not limited to the following embodiments. The present invention can be embodied by adding appropriate modification within the intended scope of the present invention.

[Method for manufacturing ceramide dispersion composition]

A method for manufacturing a ceramide dispersion composition of the present invention includes obtaining a crude dispersion liquid (hereinafter, referred to as a "crude dispersion liquid preparation step" as well) by performing a dispersion treatment in a state where a mixture (hereinafter, referred to as a "liquid before a dispersion treatment" as well), which contains ceramide, a nonionic surfactant, and a polyhydric alcohol in an amount of 10 times to 12,000 times as much as the mass of the ceramide, is heated to a temperature of equal to or higher than 100°C, and performing a high-pressure emulsification treatment (hereinafter, referred to as a "high-pressure emulsification treatment step" as appropriate) after mixing the crude dispersion liquid with water or a water-containing composition (hereinafter, referred to as "water or the like" as well).

If necessary, the manufacturing method of the present invention may include other steps.

In the manufacturing method of the present invention, through a process in which a mixture obtained by combining ceramide, a nonionic surfactant, and a polyhydric alcohol in a specific amount in advance is heated to a temperature of equal to or higher than 100°C and subjected to a dispersion treatment in a state where the ceramide is melted, and then the obtained crude dispersion liquid is mixed with water or the like and subjected to a high-pressure emulsification treatment, it is possible to obtain a fine ceramide dispersion particles in which the surface of the particles is in such a state that facilitates skin penetration.

When being used in an external preparation for skin such as cosmetics, the ceramide dispersion composition containing the fine ceramide dispersion particles exerts a skin care effect that is markedly better than that of a ceramide dispersion composition containing ceramide dispersion particles having the equivalent particle size manufactured by the method of the related art. It is unclear why the ceramide dispersion composition obtained by the manufacturing

method of the present invention exerts such an effect, but according to the inventors of the present invention, the reason is assumed to be as below.

Generally, it is considered that, for the improvement of the skin care effect of ceramide, from the viewpoint of skin penetration, the smaller the particle size of the ceramide dispersion particles, the better. In the manufacturing method of the present invention, a mixture obtained by combining ceramide, a nonionic surfactant, and a polyhydric alcohol in a specific amount in advance is heated to a temperature of equal to or higher than 100°C and subjected to a dispersion treatment in a state where the ceramide is melted, and then the obtained crude dispersion liquid is mixed with water or the like and subjected to a high-pressure emulsification treatment. Presumably, because of the above process, fine ceramide dispersion particles in which the surface of the particles is in such a state that facilitates skin penetration may be obtained.

Hereinafter, each of the steps in the manufacturing method of the present invention will be specifically described. The components or the like used in each of the steps will be specifically described later.

<Crude dispersion liquid preparation step>

The crude dispersion liquid preparation step is a step of obtaining a crude dispersion liquid by performing a dispersion treatment in a state where a mixture, which contains ceramide, a nonionic surfactant, and a polyhydric alcohol in an amount of 10 times to 12,000 times as much as the mass of the ceramide, is heated to a temperature of equal to or higher than 100°C.

In the crude dispersion liquid preparation step, it is possible to obtain a crude dispersion liquid in which ceramide dispersion particles (disperse phase) containing ceramide in a molten state by heating is coarsely dispersed in a continuous phase containing a polyhydric alcohol.

The content of the polyhydric alcohol is 10 times to 12,000 times as much as the mass of the ceramide, preferably 30 times to 1,000 times as much as the mass of the ceramide, and more preferably 50 times to 500 times as much as the mass of the ceramide.

If the content of the polyhydric alcohol is less than 10 times as much as the mass of the ceramide, it is difficult to form fine ceramide dispersion particles.

If the content of the polyhydric alcohol is greater than 12,000 times as much as the mass of the ceramide, the temporal stability of the ceramide dispersion particles deteriorates, and the skin care effect of the obtained ceramide dispersion composition becomes insufficient.

The ceramide, the nonionic surfactant, and the polyhydric alcohol should be simply mixed together. The ceramide, the nonionic surfactant, and the polyhydric alcohol may be mixed together at once, or these components may be mixed separately. The mixing method is not particularly limited, and examples thereof include mixing by stirring.

If necessary, the liquid before a dispersion treatment may contain other components in addition to the ceramide, the nonionic surfactant, and the polyhydric alcohol. Examples of other components include a component such as cholesterol, and details thereof will be described later.

In the crude dispersion liquid preparation step, from the viewpoint of melting the ceramide, the liquid before a dispersion treatment is heated to a temperature of equal to or higher than 100°C. The temperature to which the liquid before a dispersion treatment is heated is preferably equal to or higher than 100°C and equal to or lower than 200°C, and more preferably equal to or higher than 105°C and equal to or lower than 140°C. If the liquid before a dispersion treatment is heated to a temperature of higher than 140°C, the ceramide is oxidized and decomposed in some cases.

The liquid before a dispersion treatment should reach a temperature of equal to or higher than 100°C at the time when the liquid is subjected to a dispersion treatment. The liquid before a dispersion treatment may be heated to a temperature of 100°C in advance and then subjected to the dispersion treatment in a state where the liquid temperature is kept at a temperature of equal to or higher than 100°C, or may be subjected to the dispersion treatment in a state where the liquid before a dispersion treatment is being heated to a temperature of equal to or higher than 100°C.

The means for heating the liquid before a dispersion treatment to a temperature of equal to or higher than 100°C is not particularly limited, and examples thereof include a general heating device. Examples of the heating device include a thermostatic chamber and the like.

The means for obtaining a crude dispersion liquid by performing the dispersion treatment on the liquid before a dispersion treatment is not particularly limited, and examples thereof include a general stirring device. Examples of the stirring device include a magnetic stirrer, a household mixer, a paddle mixer, an impeller mixer, a homogenizer, a disper mixer, an ultramixer, and the like.

The time of the dispersion treatment is not particularly limited, and can be appropriately set according to the type of the stirring device, the composition of the mixture (liquid before a dispersion treatment), and the like.

<High-pressure emulsification treatment step>

The high-pressure emulsification treatment step is a step of performing a high-pressure emulsification treatment after mixing the crude dispersion liquid obtained by the crude dispersion liquid preparation step with water or the like.

In the high-pressure emulsification treatment step, the crude dispersion liquid of the molten ceramide is mixed with water or the like and subjected to the high-pressure emulsification treatment, thereby obtaining a ceramide dispersion composition containing ceramide dispersion particles.

From the viewpoint of preventing bumping, the temperature of the crude dispersion liquid at the time of being mixed with water or the like is preferably set to be equal to or lower than 100°C, and more preferably set to be 90°C to 100°C.

The temperature of water or the like is not particularly limited. From the viewpoint of obtaining fine ceramide dispersion particles, the temperature of water or the like is preferably set to be 40°C to 90°C, and more preferably set to be 50°C to 80°C.

The crude dispersion liquid and water or the like may be mixed together at once, or may be mixed in a manner in which one is added little by little to the other. From the viewpoint of obtaining fine ceramide dispersion particles, the mixing method for the crude dispersion liquid and water or the like is preferably a method of adding water or the like to the crude dispersion liquid being stirred. Examples of the mixing method include mixing by stirring.

The mixing ratio between the crude dispersion liquid and water or the like is not particularly limited. From the viewpoint of obtaining fine ceramide dispersion particles, the mixing ratio between the crude dispersion liquid and water or the like expressed by a ratio (based on mass) of crude dispersion liquid/water or the like is preferably 1/20 to 10/1, more preferably 1/10 to 5/1, and even more preferably 1/2 to 2/1.

From the viewpoint of obtaining fine ceramide dispersion particles and temporal stability, it is preferable that the crude dispersion liquid and water or the like are mixed such that a ratio between a disperse phase and a continuous phase, expressed as a ratio (based on mass) of disperse phase/continuous phase, in the ceramide dispersion composition obtained by the manufacturing method of the present invention preferably becomes 1/1,000 to 1/5, more preferably becomes 1/100 to 1/10, and even more preferably becomes 1/50 to 1/10.

The "high-pressure emulsification treatment" in the present invention means a dispersion treatment in which a shearing force (treatment pressure) of equal to or greater than 50 MPa is applied to a substance to be dispersed. From the viewpoint of obtaining fine ceramide dispersion particles, the shearing force applied to a substance to be dispersed is preferably equal to or greater than 100 MPa, more preferably equal to or greater than 180 MPa, and even more preferably equal to or greater than 200 MPa. In a commercially available device, from the viewpoint of temperature increase and pressure resistance, the upper limit of the shearing force applied to a substance to be dispersed is preferably equal to or less than 300 MPa.

The means for the high-pressure emulsification treatment is not particularly limited, and examples thereof include a general high-pressure emulsification device. Examples of the high-pressure emulsification device include ULTIMIZER HJP-25005 (manufactured by SUGINO MACHINE LIMITED), a microfluidizer (manufactured by MICROFLUIDICS INTERNATIONAL CORP.), NANOMIZER (manufactured by Yoshida Kikai Co., Ltd.), a Gaulin-type homogenizer (manufactured by APV), a Lannier-type homogenizer (manufactured by Lannier), and a high-pressure homogenizer such as a high-pressure homogenizer (manufactured by Niro Soavi), a homogenizer (manufactured by SANWA MACHINARY TRADING CO., LTD.), a high-pressure homogenizer (manufactured by IZUMI FOOD MACHINARY Co., Ltd.), or an ultrahigh-pressure homogenizer (manufactured by IKA).

From the viewpoint of obtaining fine ceramide dispersion particles, the temperature at the time of the high-pressure emulsification treatment is preferably set to be 20°C to 80°C, and more preferably set to be 40°C to 70°C.

The high-pressure emulsification treatment may be performed once. In order to improve the uniformity of the entirety of the liquid, the high-pressure emulsification treatment is preferably performed twice or more, and more preferably performed twice to five times.

In the high-pressure emulsification step, from the viewpoint of obtaining fine ceramide dispersion particles, after the crude dispersion liquid is mixed with water or the like, a dispersion treatment performed by applying ultrasonic waves is preferably carried out before the high-pressure emulsification treatment. In the dispersion treatment performed by applying ultrasonic waves, a general ultrasonic dispersion device can be used.

Examples of the ultrasonic dispersion device include ultrasonic homogenizers US-600, US-1200T, RUS-1200T, and MUS-1200T (all manufactured by NISSEI Corporation.), ultrasonic processors UIP2000, UIP4000, UIP8000, and UIP16000 (all manufactured by Hielscher Ultrasonics GmbH), and the like. These ultrasonic dispersion devices can be used at a frequency of equal to or less than 25 kHz, and preferably at a frequency of 15 kHz to 20 kHz.

<Other steps>

If necessary, within a scope that does not impair the effects of the present invention, the manufacturing method of the present invention may include other steps.

Examples of other steps include a heat sterilization step and the like.

Hereinafter, the components and the like used in each of the steps in the manufacturing method of the present invention will be specifically described.

(Ceramide)

The ceramide in the present invention includes ceramide and a derivative thereof, and the source thereof is not limited regardless of synthetic ceramide and extracted ceramide. In the present invention "ceramide" includes natural type ceramide, which will be described later, compounds having natural type ceramide as a basic skeleton, and precursor substances that can be derived from those compounds, and is used as a generic name for natural type ceramide, sugar-modified ceramide such as glycosphingolipid, and synthetic ceramide.

Hereinafter, natural type ceramide, sugar-modified ceramide, and synthetic ceramide will be specifically described in this order.

-Natural type ceramide-

In the present specification, the "natural type ceramide" means ceramide having the same structure as the structure of ceramide present in the stratum corneum of human skin.

As the ceramide in the present invention, according to the purpose, it is also possible to use modified natural type ceramide obtained by introducing a double bond into the molecule so as to impart solubility or by introducing a hydrophobic group so as to impart penetrating property.

The ceramide called natural type having a general structure may be a natural substance (extract), ceramide obtained by a microbial fermentation method, a synthetic substance, or ceramide derived from animal.

In the present invention, as ceramide, natural type (D (-) isomer) optically active ceramide or non-natural type (L (+) isomer) optically active ceramide may be used. Furthermore, a mixture of a natural type optically active ceramide and a non-natural type optically active ceramide may be used. The relative steric configuration of the ceramide may be steric configuration of a natural type, steric configuration of a non-natural type, or a steric configuration of a mixture of a natural type and a non-natural type.

In a case where the ceramide dispersion composition obtained by the manufacturing method of the present invention is used as an emollient or the like for skin, from the viewpoint of a barrier effect, it is preferable to use more natural-type ceramide than non-natural type ceramide.

Examples of the natural type ceramide that can be suitably used as ceramide in the present invention include Ceramide 1, Ceramide 4, Ceramide 9, Ceramide 2, Ceramide 3, Ceramide 5, Ceramide 6, Ceramide 7, Ceramide 8, and the like.

The above natural type ceramide can be obtained as a commercial product, and examples thereof include CERAMIDE I, CERAMIDE EOP27, CERAMIDE EOS27, CERAMIDE III, CERAMIDE IIIA, CERAMIDE IIIB, CERAMIDE IIIC, and CERAMIDE VI (all trade names, manufactured by Evonik Industries AG); CERAMIDE TIC-001 (trade name, manufactured by Takasago International Corporation); CERAMIDE II (trade name, manufactured by Quest International); DS-CERAMIDE VI, DS-CLA-PHYTOCERAMIDE, C6-PHYTOCERAMIDE, and DS-CERAMIDE Y3S (all trade names, manufactured by Doosan Corporation); CERAMIDE 2 (manufactured by Sederma); and the like.

(Sugar-modified ceramide)

The sugar-modified ceramide is ceramide containing saccharide in a molecule. Examples of the saccharide contained in the ceramide molecule include monosaccharide such as glucose and galactose, disaccharide such as lactose and maltose, oligosaccharide obtained by polymerizing at least one kind of saccharide selected from the group consisting of monosaccharide and disaccharide through a glucoside bond, polysaccharide, and the like. Furthermore, the saccharide may be a sugar derivative obtained by substituting a hydroxyl group in a sugar unit with other groups. Examples of the sugar derivative include glucosamine, glucuronic acid, N-acetylglucosamine, and the like. Among these, from the viewpoint of the dispersion stability of the ceramide dispersion particles, as the saccharide, saccharide having 1 to 5 sugar units is preferable, at least one kind of saccharide selected from glucose and lactose is more preferable, and glucose is even more preferable.

Specific examples of the sugar-modified ceramide include the following sugar-modified ceramides.

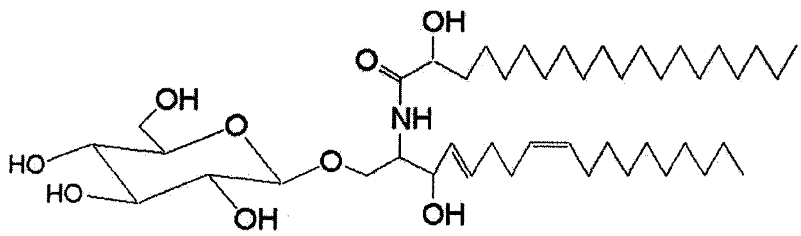

(1-1)

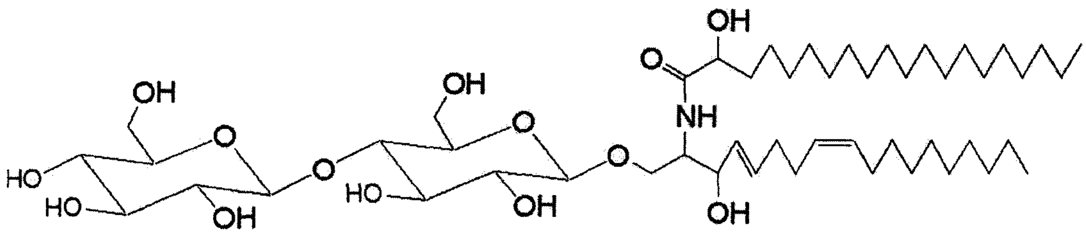

(1-2)

The sugar-modified ceramide may be obtained by synthesis or may be obtained as a commercially available product. For example, the above example compound (1-1) can be obtained as "RICE SPHINGOGLYCOLIPID" (trade name, OKAYASU SHOTEN CO., LTD.).

(Synthetic ceramide)

The synthetic ceramide is a compound synthesized by imitating the structure of ceramide. Examples of known compounds of synthetic ceramide include synthetic ceramides (example compounds (2-1) and (2-2)) shown in the following structural formulae.

(2-1)

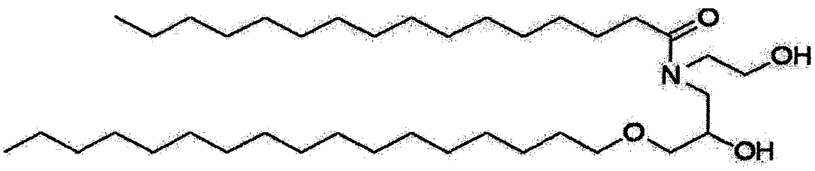

(2-2)

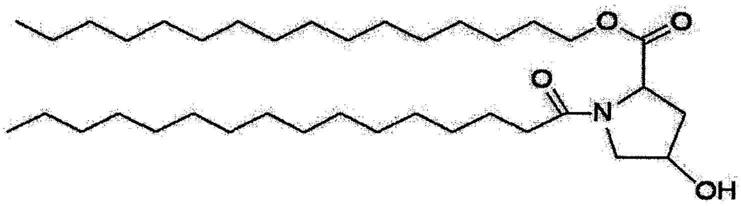

In a case where synthetic ceramide is used as ceramide in the manufacturing method of the present invention, the ceramide is preferably synthetic ceramide synthesized by imitating the structure of the natural type ceramide or the structure of the sugar-modified ceramide, and more preferably synthetic ceramide synthesized by imitating the structure of the natural type ceramide. The synthetic ceramide can be obtained as a commercially available product. For example, the above example compound (2-2) (cetylhydroxyproline palmitamide) can be obtained as "CERAMIDE BIO" (trade name, manufactured by Symrise AG).

In the manufacturing method of the present invention, the ceramide is preferably a compound represented by the following Formula (I) among the aforementioned ceramides, because then a ceramide dispersion composition, which contain finer ceramide dispersion particles and exhibit a markedly excellent skin care effect in a case where the composition is used in an external preparation for skin, can be obtained.

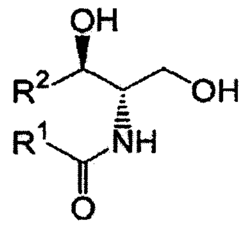 • • • (I)

(In Formula (I), $R^1$ and $R^2$ have the same definition as described above.)

Hereinafter, Formula (I) will be described.

The total number of carbon atoms of the hydrocarbon group represented by $R^1$ is 40 to 55, preferably 42 to 52, and more preferably 45 to 48.

The number of ester bonds that the hydrocarbon group represented by $R^1$ contains in a carbon chain is preferably 1 or 2 or greater, and more preferably 1.

Examples of the multiple bond that the hydrocarbon group represented by $R^1$ may contain in a carbon chain include a carbon-carbon double bond or a carbon-carbon triple bond, and among these, a carbon-carbon double bond is preferable.

Examples of the aromatic ring that the hydrocarbon group represented by $R^1$ may contain in a carbon chain include a benzene ring, a naphthalene ring, an anthracene ring, and the like.

Examples of the aromatic heterocyclic ring that the hydrocarbon group represented by $R^1$ may contain in a carbon chain include a pyridine ring, a pyrazine ring, and the like.

The hydrocarbon group represented by $R^1$ may have a structure in which a saturated or unsaturated hydrocarbon group having 25 to 30 carbon atoms is linked to a saturated or unsaturated hydrocarbon group having 16 to 18 carbon atoms through a single ester bond.

Preferred examples of the hydrocarbon group represented by $R^1$ include a hydrocarbon group which has 40 to 55 carbon atoms in total and has a structure in which a linear fatty acid such as linoleic acid or stearic acid is linked to the ω terminal of a saturated linear ω-hydroxy fatty acid through an ester bond.

The hydrocarbon group represented by $R^1$ may further have a substituent, and examples of the substituent include a carbonyl group, an oxy group, an amide group, and the like.

A specific example of $R^1$ will be shown below, but $R^1$ is not limited to the following specific example.

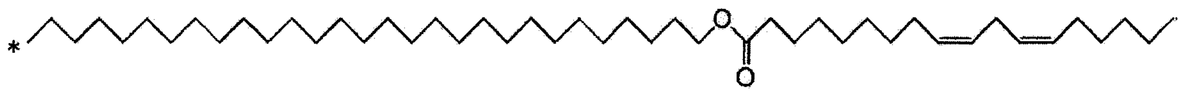

* represents a linking portion.

The number of carbon atoms contained in the alkyl group represented by $R^2$ is 11 to 21, preferably 13 to 21, and more preferably 14 to 20. The alkyl group represented by $R^2$ may be linear or branched, and is preferably linear.

The number of carbon atoms contained in the alkenyl group represented by $R^2$ is 11 to 21, preferably 13 to 21, and more preferably 14 to 20. The alkenyl group represented by $R^2$ may be linear or branched, and is preferably linear.

The alkyl group or the alkenyl group represented by $R^2$ may have one hydroxyl group or two or more hydroxyl groups, and preferably has one hydroxyl group or two hydroxyl groups.

The hydrocarbon group represented by $R^2$ may further have a substituent, and examples of the substituent include a carbonyl group, an oxy group, an amide group, and the like.

The preferred combination of $R^1$ and $R^2$ in Formula (I) includes the combination of the preferred aspect of $R^1$ and $R^2$ described above.

Specific examples of $R^2$ will be shown below, but $R^2$ is not limited to the following specific examples.

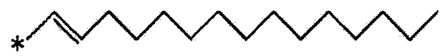

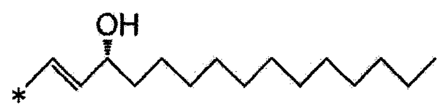

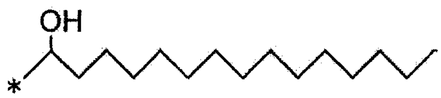

* represents a linking portion.

Specific examples of the compound represented by Formula (I) will be shown below, but the compound represented by Formula (I) is not limited to the following specific examples.

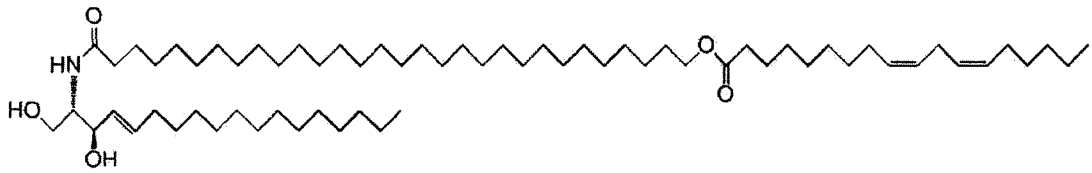

(I-1)

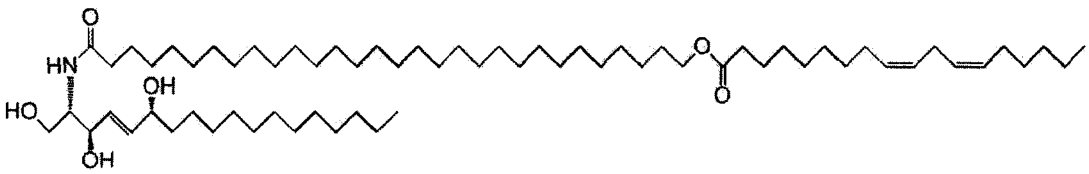

(I-2)

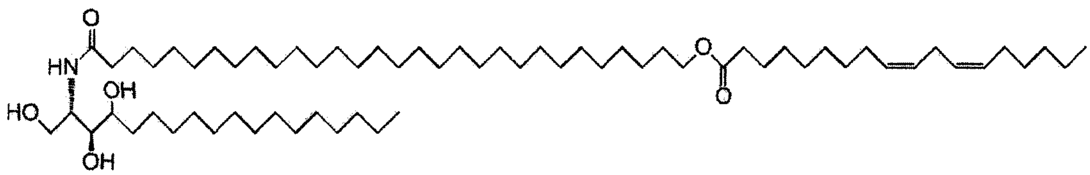

(I-3)

(I-4)

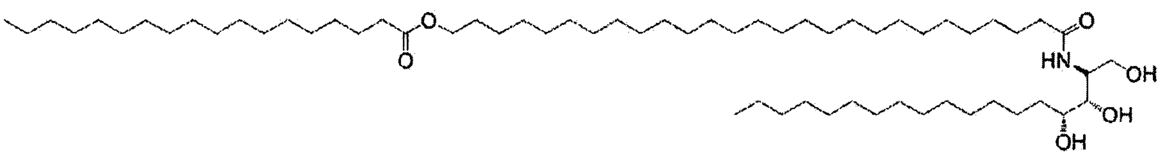

(I-5)

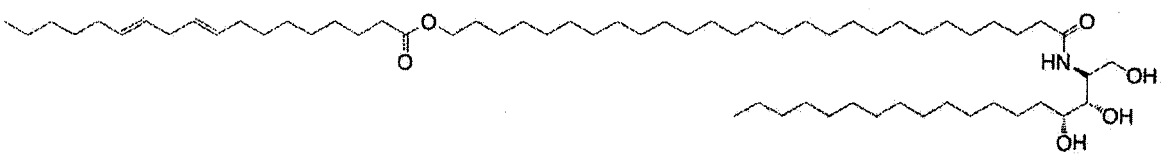

(I-6)

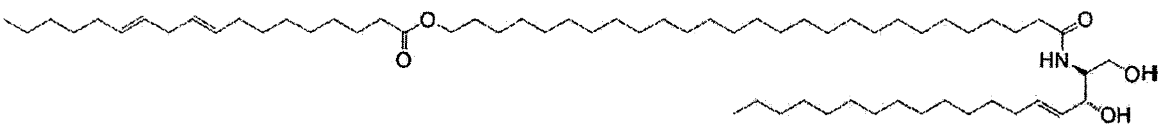

Specific examples of the compound represented by Formula (I) include the natural type ceramide such as Ceramide 1, Ceramide 4, and Ceramide 9.

If the compound represented by Formula (I) is used as ceramide in the manufacturing method of the present invention, it is possible to obtain a ceramide dispersion composition which contain finer ceramide dispersion particles and exhibit an excellent skin care effect in a case of where the composition is used in an external preparation for skin. It is unclear why such a ceramide dispersion composition is obtained, but according to the inventors of the present invention, the reason is assumed to be as below. Presumably, because the compound represented by Formula (I) has

a long hydrophobic group, exhibits strong interaction between ceramide molecules, and has a structure stable in the dispersion particles, the dispersion particles are not easily aggregated, and the fine particle size is maintained.

In the present invention, the liquid before a dispersion treatment may contain one kind of ceramide singly or two or more kinds thereof in combination. Generally, ceramide has a high melting point and high crystallinity. Therefore, from the viewpoint of emulsion stability and handleability, it is preferable to use two or more kinds of ceramide in combination.

From the viewpoint of efficient percutaneous absorption of the ceramide in a case where the obtained ceramide dispersion composition is used in an external preparation for skin and from the viewpoint of bringing about the skin care effect resulting from the ceramide, the content of the ceramide in the liquid before a dispersion treatment is preferably 0.1% by mass to 80% by mass, more preferably 1% by mass to 50% by mass, and even more preferably 2% by mass to 30% by mass, with respect to the total mass of the components contained in the ceramide disperiosn particles.

Furthermore, from the viewpoint of bringing about the skin care effect, the content of the ceramide in the liquid before a dispersion treatment is preferably 0.00001% by mass to 10% by mass, more preferably 0.00003% by mass to 5% by mass, and even more preferably 0.0001% by mass to 1% by mass, with respect to the total mass of the obtained ceramide dispersion composition.

(Nonionic surfactant)

As the nonionic surfactant in the present invention, known nonionic surfactants can be used without particular limitation. Examples of the nonionic surfactant include a glycerin fatty acid ester, polyglycerin fatty acid ester, organic acid monoglyceride, a propylene glycol fatty acid ester, a polyglycerin condensed ricinoleic acid ester, a sorbitan fatty acid ester, a sucrose fatty acid ester, a polyoxyethylene sorbitan fatty acid ester, and the like. These nonionic surfactants do not need to be nonionic surfactants highly purified by distillation or the like and may be a reaction mixture.

Among the above examples, from the viewpoint of obtaining fine ceramide dispersion particles and the dispersion stability thereof, a polyglycerin fatty acid ester is preferable as the nonionic surfactant, and a polyglycerin fatty acid ester having an HLB value of equal to or greater than 10 and equal to or less than 16 is more preferable. The polyglycerin fatty acid ester is preferable because it can greatly reduce the surface tension of disperse phase/continuous phase, and hence finer ceramide dispersion particles can be obtained.

HLB means the hydrophilic-hydrophobic balance used generally in the field of surfactants. For calculating HLB, it is possible to use a generally used calculation formula such as a Kawakami's formula. In the present invention, the following Kawakami's formula is adopted as a calculation formula for HLB.

$$HLB = 7 + 11.7 \log(Mw/Mo)$$

Mw represents the molecular weight of a hydrophilic group, and Mo represents the molecular weight of a hydrophobic group.

The numerical values of HLB listed in a catalog or the like may also be adopted. As is evident from the above formula, by using the additivity of HLB, a surfactant having any HLB value can be obtained.

It is preferable that at least one of the polyglycerin fatty acid esters is an ester of polyglycerin having an average degree of polymerization of 10 and a fatty acid having 8 to 18 carbon atoms selected from the group consisting of, for example, caprylic acid, capric acid, lauric acid, myristic acid, palmitic acid, stearic acid, oleic acid, and linoleic acid.

More preferred examples of the polyglycerin fatty acid ester include a hexaglycerin monooleic acid ester, a hexaglycerin monopalmitic acid ester, a hexaglycerin monomyristic acid ester, a hexaglycerin monolauric acid ester, a decaglycerin monolinoleic acid ester, a decaglycerin monooleic acid ester, a decaglycerine monostearic acid ester, a decaglycerin monopalmitic acid ester, a decaglycerine monomyristic acid ester, a decaglycerin monolauric acid ester, and the like. All of these polyglycerin fatty acid ester have an HLB value of equal to or greater than 10 and equal to or less than 16.

Among the above, as the polyglycerin fatty acid ester, at least one kind of compound selected from the group consisting of a decaglycerin monolinoleic acid ester (HLB = 12), a decaglycerin monooleic acid ester (HLB = 12), a decaglycerin monostearic acid ester (HLB = 12), a decaglycerin monopalmitic acid ester (HLB = 13), a decaglycerin monomyristic acid ester (HLB = 14), and a decaglycerin monolauric acid ester (HLB = 16) is more preferable, and a decaglycerin monomyristic acid ester is particularly preferable.

In the crude dispersion liquid preparation step, one or more kinds of compound selected from polyglycerin fatty acid esters having an HLB value of equal to or greater than 10 and equal to or less than 16 may be used in combination with one or more kinds of compound selected from polyglycerin fatty acid esters having an HLB value of equal to or greater than 5 and equal to or less than 15 that have a molecular structure different from the molecular structure of the

above polyglycerin fatty acid esters. The polyglycerin fatty acid esters having an HLB value of equal to or greater than 5 and equal to or less than 15 may be polyglycerin fatty acid esters included in the aforementioned polyglycerin fatty acid esters or may be other polyglycerin fatty acid esters.

As the polyglycerin fatty acid esters, commercially available products can be used.

Examples of the commercially available products of the polyglycerin fatty acid esters include NIKKOL DGMS, NIKKOL DGMO-CV, NIKKOL DGMO-90V, NIKKOL DGDO, NIKKOL DGMIS, NIKKOL DGTIS, NIKKOL TETRAGLYN 1-SV, NIKKOL TETRAGLYN 1-O, NIKKOL TETRAGLYN 3-S, NIKKOL TETRAGLYN 5-S, NIKKOL TETRAGLYN 5-O, NIKKOL HEXAGLYN 1-L, NIKKOL HEXAGLYN 1-M, NIKKOL HEXAGLYN 1-SV, NIKKOL HEXAGLYN 1-O, NIKKOL HEXAGLYN 3-S, NIKKOL HEXAGLYN 4-B, NIKKOL HEXAGLYN 5-S, NIKKOL HEXAGLYN 5-O, NIKKOL HEXAGLYN PR-15, NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, NIKKOL DECAGLYN 2-SV, NIKKOL DECAGLYN 2-ISV, NIKKOL DECAGLYN 3-SV, NIKKOL DECAGLYN 3-OV, NIKKOL DECAGLYN 5-SV, NIKKOL DECAGLYN 5-HS, NIKKOL DECAGLYN 5-IS, NIKKOL DECAGLYN 5-OV, NIKKOL DECAGLYN 5-O-R, NIKKOL DECAGLYN 7-S, NIKKOL DECAGLYN 7-O, NIKKOL DECAGLYN 10-SV, NIKKOL DECAGLYN 10-IS, NIKKOL DECAGLYN 10-OV, NIKKOL DECAGLYN 10-MAC, and NIKKOL DECAGLYN PR-20 (all trade names) manufactured by Nikko Chemicals Co., Ltd.; RYOTO POLYGLYESTER L-7D, L-10D, M-10D, P-8D, SWA-10D, SWA-15D, SWA-20D, S-24D, S-28D, O-15D, O-50D, B-70D, B-100D, ER-60D, LOP-120DP, DS13W, DS3, HS11, HS9, TS4, TS2, DL15, and DO13 (all trade names) manufactured by Mitsubishi-Kagaku Foods Corporation; SUNSOFT Q-17UL, SUNSOFT Q-14S, and SUNSOFT A-141C (all trade names) manufactured by Taiyo Kagaku Co., Ltd.; POEM DO-100 and POEM J-0021 (all trade names) manufactured by RIKEN VITAMIN; and the like.

Among the above, NIKKOL DECAGLYN 1-L, NIKKOL DECAGLYN 1-M, NIKKOL DECAGLYN 1-SV, NIKKOL DECAGLYN 1-50SV, NIKKOL DECAGLYN 1-ISV, NIKKOL DECAGLYN 1-O, NIKKOL DECAGLYN 1-OV, NIKKOL DECAGLYN 1-LN, RYOTOPOLYGLYESTER L-7D, L-10D, M-10D, P-8D, SWA-10D, SWA-15D, SWA-20D, S-24D, S-28D, O-15D, O-50D, B-70D, B-100D, ER-60D, and LOP-120DP are preferable as the commercially available product of the polyglycerin fatty acid ester.

The liquid before a dispersion treatment in the present invention may contain one kind of nonionic surfactant singly or contain two or more kinds thereof in combination.

The content of the nonionic surfactant in the liquid before a dispersion treatment is preferably 1 time to 70 times as much as the mass of the ceramide, more preferably 3 times to 40 times as much as the mass of the ceramide, and even more preferably 5 times to 20 times as much as the mass of the ceramide.

If the content of the nonionic surfactant in the liquid before a dispersion treatment is equal to or greater than 1% by mass with respect to the mass of the ceramide, it is possible to form finer ceramide dispersion particles.

If the content of the nonionic surfactant in the liquid before a dispersion treatment is equal to or less than 70% by mass with respect to the mass of the ceramide, it is possible to form ceramide dispersion particles having excellent dispersion stability.

(Polyhydric alcohol)

The polyhydric alcohol in the present invention is not particularly limited as long as it is an alcohol having two or more hydroxyl groups, and known polyhydric alcohols can be used.

Examples of the polyhydric alcohol include glycerin, diglycerin, triglycerin, polyglycerin, a diol compound (for example, 1,3-butylene glycol (1,3-BG), isoprene glycol, polyethylene glycol, propylene glycol, polypropylene glycol, ethylene glycol, diethylene glycol, neopentyl glycol, 1,2-pentanediol, 1,2-hexanediol, dipropylene glycol, or 3-methyl-1,3-butanediol), maltitol, reduced sugar syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, sugar obtained by amylolysis, a reduced sugar alcohol obtained by amylolysis, and the like.

Among the above examples, from the viewpoint of obtaining fine ceramide dispersion particles and improving the skin care effect, at least one kind of polyhydric alcohol selected from the group consisting of glycerin and a diol compound is preferable as the polyhydric alcohol, and glycerin and at least one kind of diol compound are more preferable. Particularly, if glycerin and at least one kind of diol compound are used in combination as the polyhydric alcohol, it is possible to obtain a ceramide dispersion composition which contains finer ceramide dispersion particles and has a markedly excellent skin care effect.

In the present invention, the "diol compound" means a compound containing two hydroxy groups in a molecule.

In a case where glycerin and at least one kind of diol compound are used in combination, from the viewpoint of the temporal stability of the ceramide dispersion composition, a ratio between the glycerin and the diol compound (amount of glycerin:total amount of diol compound) is preferably 100:1 to 1:1, more preferably 75:1 to 5:1, and even more preferably 50:1 to 10:1, based on mass.

From the viewpoint of making finer ceramide dispersion particles and the temporal stability thereof, the diol

compound is preferably at least one kind of compound selected from the group consisting of 1,3-butylene glycol, dipropylene glycol, 1,4-butylene glycol, diethylene glycol, ethylene glycol, propylene glycol, 1,2-pentanediol, 1,5-pentanediol, 1,4-pentanediol, 2,4-pentanediol, 1,2-hexanediol, and 1,6-hexanediol, more preferably at least one kind of compound selected from the group consisting of 1,3-butylene glycol and dipropylene glycol, and even more preferably 1,3-butylene glycol.

(Water or water-containing composition)

The water or the water-containing composition in the present invention is a continuous phase in which the ceramide dispersion particles are dispersed as a disperse phase.
The water in the present invention is preferably water containing a small amount of impurities, such as pure water or deionized water.
In the present invention, the "water-containing composition" means that the composition should contain water. The components other than water contained in the composition are not particularly limited, and examples thereof include known water-soluble components that can be generally added to a continuous phase in manufacturing a dispersion composition.
Examples of the components other than water contained in the composition include lecithin and the like.
The amount of water mixed with the crude dispersion liquid is not particularly limited, and can be appropriately set according to the intended concentration as desired.
From the viewpoint of obtaining fine ceramide dispersion particles, the amount of water mixed with the crude dispersion liquid (in a case where the crude dispersion liquid is mixed with the water-containing composition, the amount of water contained in the composition) expressed as a ratio (based on mass) of crude dispersion liquid/water or the like is preferably 1/20 to 10/1, more preferably 1/10 to 5/1, and even more preferably 1/2 to 2/1.

(Lecithin)

In the manufacturing method of the present invention, it is preferable to use lecithin.
If lecithin is used in the manufacturing method of the present invention, it is possible to obtain a ceramide dispersion composition containing finer ceramide dispersion particles.
In a case where lecithin is used in the manufacturing method of the present invention, lecithin may be used in any of the crude dispersion liquid preparation step and the high-pressure emulsification treatment step. However, from the viewpoint of obtaining fine ceramide dispersion particles, it is preferable to use lecithin in the high-pressure emulsification treatment step.
Lecithin has a hydrophilic group and a hydrophobic group in a molecule. Therefore, in the related art, lecithin is widely used as an emulsifier in the field of foods, pharmaceutical products, cosmetics, and the like.
Industrially, lecithin with purity of equal to or higher than 60% is used, and in the manufacturing method of the present invention, the lecithin with purity of equal to or higher than 60% can also be used. From the viewpoint of forming finer ceramide dispersion particles, lecithin with purity of equal to or higher than 80% that is generally called high-purity lecithin is preferable, and lecithin with purity of equal to or higher than 90% is more preferable.
Examples of lecithin include various types of known lecithin extracted and separated from the biological body of plants, animals, and microorganisms.
Specific examples of lecithin include various types of lecithin derived from plants such as soybean, corn, peanut, rape seeds, and barley, egg yolk, animals such as cows, and microorganisms such as E. coli.
Examples of lecithin compounds include, based on chemical nomenclature, glycerolecithin such as phosphatidic acid, phosphatidylglycerin, phosphatidylinositol, phosphatidyl ethanolamine, phosphatidyl methylethanolamine, phosphatidylcholine, phosphaditylserine, bis-phosphatidic acid, and diphosphatidylglycerin (cardiolipin); sphingolecithin such as sphingomyelin; and the like.
In the manufacturing method of the present invention, in addition to the above high-purity lecithin, hydrogenated lecithin, enzymatically decomposed lecithin, enzymatically decomposed hydrogenated lecithin, hydroxy lecithin, and the like can be used. In the manufacturing method of the present invention, lecithin can be used singly or used in the form of a mixture of a plurality of kinds of lecithin.
In a case where lecithin is used in the manufacturing method of the present invention, the amount of lecithin used is preferably 0.01 times to 20 times as much as the mass of the nonionic surfactant, more preferably 0.1 times to 10 times as much as the mass of the nonionic surfactant, and even more preferably 0.4 times to 5 times as much as the mass of the nonionic surfactant.

(Cholesterol)

In the manufacturing method of the present invention, cholesterol may be used.

It is preferable to use cholesterol in the manufacturing method of the present invention because then a ceramide dispersion composition, which exhibits a better skin care effect in a case where the composition is used in an external preparation for skin, can be obtained.

In a case where cholesterol is used in the manufacturing method of the present invention, the cholesterol may be used in any of the crude dispersion liquid preparation step and the high-pressure emulsification treatment step. From the viewpoint of obtaining fine ceramide dispersion particles, it is preferable to use cholesterol in the crude dispersion liquid preparation step.

As cholesterol, it is possible to preferably use cholesterol which contains lanolin as a main component and is prepared by purifying crude cholesterol obtained by extraction. As the cholesterol, a commercially available product may be used. Examples of the commercially available cholesterol product include "CHOLESTEROL JSQI (trade name)" manufactured by Nippon Fine Chemical, "CHOLESTEROL" manufactured by Croda Japan KK., and the like.

In a case where cholesterol is used in the manufacturing method of the present invention, the amount of cholesterol used is preferably 0.00001% by mass to 10% by mass, more preferably 0.00003% by mass to 5% by mass, and even more preferably 0.0001% by mass to 1% by mass, with respect to the total mass of the components used in the manufacturing method of the present invention.

(Other components)

In the manufacturing method of the present invention, if necessary, components other than the aforementioned components can be used within a scope that does not impair the effects of the present invention.

Examples of other components include a component which can be generally added in manufacturing a dispersion composition, a component which is used if necessary according to the use of the obtained ceramide dispersion composition, and the like.

In a case where the ceramide dispersion composition is used as an external preparation for skin, examples of the aforementioned other components include various medicinal components, a preservative, a germicide, a colorant, a cooling agent such as menthol or camphor, a plant extract, a pH buffering agent, an antioxidant, an ultraviolet absorber, an ultraviolet scattering agent, a fragrance, and the like.

Those other components can be used in at least one step selected from the group consisting of the crude dispersion liquid preparation step, the high-pressure emulsification treatment step, and other steps, according to the function thereof.

(Oleaginous component staying liquid form at 25°C)

The ceramide dispersion composition obtained by the manufacturing method of the present invention preferably does not contain an oleaginous component staying in a liquid form at 25°C or contains an oleaginous component staying in a liquid form at 25°C in an amount of equal to or less than 1% by mass, and more preferably does not contain an oleaginous component staying in a liquid form at 25°C.

In the high-pressure emulsification method of the related art, an oleaginous component staying in a liquid form at 25°C, such as squalane, is generally used. Accordingly, the obtained dispersion composition generally contains the oleaginous component staying in a liquid form at 25°C.

In the manufacturing method of the present invention, the obtained ceramide dispersion composition is caused not to contain the oleaginous component staying in a liquid form at 25°C, or even in a case where the composition contains the oleaginous component, the content is controlled to be equal to or less than 1% by mass. Alternatively, the oleaginous component staying in a liquid form at 25°C is not used, or even in a case where the oleaginous component is used, the amount thereof used is controlled. In this way, it is possible to obtain a ceramide dispersion composition which contain finer ceramide dispersion particles and exhibits a better skin care effect in a case where the composition is used as in an external preparation for skin.

In the present invention, "staying in a liquid form at 25°C" means that the melting point or the softening point is lower than 25°C. Furthermore, in the present invention, the "oleaginous component" means a component which has solubility of less than 0.1% by mass in water at 25°C and is generally used as an oleaginous component in the field of cosmetics, pharmaceutical products, foods, and the like.

Specific examples of the oleaginous component staying in a liquid form at 25°C include hydrocarbon oil such as squalane and liquid paraffin; silicone oil such as dimethyl polysiloxane, dimethylcyclopolysiloxane, methyl phenyl polysiloxane, methyl hydrogen polysiloxane, and higher alcohol-modified organopolysiloxane; fluorine oil such as fluoropolyether and perfluoroalkyl ether silicone; vegetable oil such as olive oil and jojoba oil; animal oil such as liquid lanolin;

fatty acid esters such as diisostearyl malate, octyldodecyl lactate, isotridecyl isononanoate, isopropyl isostearate, and octyldodecyl myristate; ester oil composed of a fatty acid and a polyhydric alcohol, such as neopentyl glycol dicaprylate; ester oil such as a glycerin derivative and an amino acid derivative; and the like.

[Ceramide dispersion composition]

(Particle size of ceramide dispersion particles)

The ceramide dispersion composition obtained by the manufacturing method of the present invention is an oil-in-water type emulsion in which ceramide dispersion particles are dispersed as a disperse phase in a continuous phase. According to the manufacturing method of the present invention, it is possible to obtain a ceramide dispersion composition containing ceramide dispersion particles having an average particle size of less than 60 nm. Furthermore, according to the manufacturing method of the present invention, it is possible to obtain ceramide dispersion particles preferably having an average particle size of less than 40 nm, more preferably having an average particle size of less than 30 nm, and even more preferably having an average particle size of less than 20 nm. The lower limit of the average particle size of the ceramide dispersion particles is not particularly limited, and the average particle size of the ceramide dispersion particles can be set to be, for example, equal to or greater than 1 nm.

In the present invention, the "average particle size of ceramide dispersion particles" means a volume average particle size of the ceramide dispersion particles present in the ceramide dispersion composition.

From the viewpoint of accuracy and simplicity of measurement, it is preferable to measure the volume average particle size of the ceramide dispersion particles by using a dynamic light scattering method.

Examples of commercially available measurement devices using the dynamic light scattering method include a dynamic light scattering-type nanotrac particle size analyzer UPA (manufactured by NIKKISO CO., LTD.), a dynamic light scattering-type particle size distribution analyzer LB-550 (manufactured by HORIBA, LTD.), a concentrated system particle size analyzer FPAR-1000 (OTSUKA ELECTRONICS CO., LTD.), and the like.

In the present specification, the volume average particle size of the ceramide dispersion particles is a value measured using the dynamic light scattering-type nanotrac particle size analyzer UPA (manufactured by NIKKISO CO., LTD.), and specifically, the value is measured as below. In the present invention, the volume average particle size of the ceramide dispersion particles can also be measured using other analyzers.

The volume average particle size of the ceramide dispersion particles is measured using a quartz cell by diluting the ceramide dispersion composition obtained by the manufacturing method of the present invention with pure water such that the concentration of ceramide contained in a sample separated from the composition becomes 0.03% by mass. The volume average particle size can be determined as a volume average particle size (Mv) which is obtained in a case where a refractive index of a sample is to be 1.600, a refractive index of a dispersion medium is to be 1.333 (pure water), and the viscosity of pure water is set as the viscosity of the dispersion medium.

(Use of ceramide dispersion composition)

The ceramide dispersion composition obtained by the manufacturing method of the present invention exhibits an excellent skin care effect in a case where the composition is used in an external preparation for skin such as cosmetics. Therefore, the ceramide dispersion composition can be widely used directly as a pharmaceutical product (an external preparation or a dermal product), cosmetics, a cleanser, and the like or used as a component of these. Examples of the pharmaceutical product include a parenteral agent such as an ointment. Examples of the cosmetics include a toner, a serum, a gel, an emulsion, a hair conditioner, a hair treatment, a rinse, and the like. Examples of the cleanser include a facial cleanser, a body soap, a shampoo, and the like. Here, the use of the ceramide dispersion composition of the present invention is not limited to these.

In a case where the ceramide dispersion composition obtained by the manufacturing method of the present invention is used in pharmaceutical products or cosmetics, if necessary, components that can be added to pharmaceutical products and cosmetics can be appropriately added thereto.

In a case where the ceramide dispersion composition obtained by the manufacturing method of the present invention is used in cosmetics, the composition may take any form of formulation that is generally known as cosmetics such as a toner, a serum, a gel, an emulsion, a hair conditioner, a hair treatment, and a rinse. In a case where the ceramide dispersion composition obtained by the manufacturing method of the present invention is used in cosmetics, from the viewpoint of exploiting the characteristics of the ceramide dispersion particles as fine particles, the ceramide dispersion composition preferably takes the form of formulation having high transparency, for example, a toner, a serum, or a gel preparation. Examples

Hereinafter, the present invention will be more specifically described based on examples, but the present invention is not limited to the following examples as long as the gist of the present invention is maintained. Herein, unless

otherwise specified, "part" is based on mass.

[Example 1]

39.5 g of a liquid A composed as below was missed stirred and mixed for 10 minutes at 110°C, thereby obtaining a crude dispersion liquid. The obtained crude dispersion liquid was cooled to 100°C, a liquid B obtained by dissolving the components composed as below at 70°C was added thereto, and then the resultant was dispersed for 3 minutes using an ultrasonic homogenizer US-600 (manufactured by NISSEI Corporation.), thereby obtaining a preliminary dispersion. Subsequently, the obtained preliminary dispersion was cooled to 60°C and then subjected to high-pressure emulsification (dispersion) treatment for 5 minutes at a pressure of 245 MPa by using ULTIMAIZER HJP-25005 (manufactured by SUGINO MACHINE LIMITED), thereby obtaining a ceramide dispersion composition of Example 1.
The details of each component used in the liquids A and B are as below.
[Liquid A]

| | |
|---|---|
| •Ceramide I | 0.3 parts |
| •Cholesterol | 0.3 parts |
| •1,3-Butylene glycol | 0.9 parts |
| •Decaglycerin monomyristic acid ester | 3.0 parts |
| •Glycerin | 35.0 parts |

[Liquid B]

| | |
|---|---|
| •Lecithin | 4.0 parts |
| •Iodopropynyl butylcarbamate | 0.01 parts |
| •Water | 56.5 parts |

[Examples 2 to 12 and 14 to 16 and Comparative examples 1 to 3, 7, and 8]

Ceramide dispersion compositions of Examples 2 to 12 and 14 to 16 and Comparative examples 1 to 3, 7, and 8 were obtained in the same manner as in Example 1, except that the liquids A and B composed as shown in Tables 1, 2, and 3 were used.

[Example 13]

A ceramide dispersion composition of Example 13 was obtained in the same manner as in Example 1, except that the high-pressure emulsification treatment was performed at 50 MPa.

[Comparative example 4]

A ceramide dispersion composition of Comparative example 4 was obtained in the same manner as in Example 1, except that a crude dispersion liquid was obtained by stirring and mixing the liquid A at 80°C.

[Comparative example 5]

A ceramide dispersion composition of Comparative example 5 was obtained in the same manner as in Example 4, except that a crude dispersion liquid was obtained by stirring and mixing the liquid A at 80°C.

[Comparative example 6]

A ceramide dispersion composition of Comparative example 6 was obtained in the same manner as in Example 6, except that a crude dispersion liquid was obtained by stirring and mixing the liquid A at 80°C.

[Evaluation]

For each of the obtained ceramide dispersion compositions of Examples 1 to 16 and Comparative examples 1 to 8, (1) particle size of the ceramide dispersion particles in the just prepared ceramide dispersion composition was

measured, and (2) skin care effect were evaluated. The results are shown in Tables 1, 2, and 3.

(1) Particle size of ceramide dispersion particles in ceramide dispersion composition

The particle size (volume average particle size) of the ceramide dispersion particles in the just prepared ceramide dispersion composition was measured using a dynamic light scattering-type nanotrac particle size analyzer UPA (manufactured by NIKKISO CO., LTD.). The volume average particle size was measured by diluting the ceramide dispersion composition with pure water such that the concentration of ceramide contained in a sample separated from the ceramide dispersion composition became 0.03% by mass. The volume average particle was determined as a volume average particle size (Mv) which was obtained in a case where a refractive index of a sample is set to be 1.600, a refractive index of a dispersion medium is set to be 1.333 (pure water), and the viscosity of pure water is set as the viscosity of the dispersion medium.

(2) Skin care effect

In December, the ceramide dispersion composition was applied to the back of left hands of five test subjects having noticeable rough skin on their hands in winter, twice a day for two weeks. The left hands were compared to the right hands to which the composition was not applied, and by giving 1 point in a case of "no change" and giving 5 points in a case of "no rough skin recognized", the degree of improvement after two weeks was evaluated into five levels. The points the five test subjects awarded were averaged.

[Table 1]

| | | | Example 1 | Example 2 | Example 3 | Example 4 | Example 5 | Example 6 |
|---|---|---|---|---|---|---|---|---|
| Formulation (part) | Liquid A | Ceramide I | 0.3 | - | - | - | - | - |
| | | Ceramide EOP | - | 0.3 | - | - | - | - |
| | | Ceramide EOS | - | - | 0.3 | - | - | - |
| | | Ceramide II | - | - | - | 0.3 | - | - |
| | | Ceramide III | - | - | - | - | 0.3 | - |
| | | Ceramide VI | - | - | - | - | - | 0.3 |
| | | Cholesterol | 0.3 | 0.3 | 0.3 | 0.3 | 0.3 | 0.3 |
| | | 1,3-butylene glycol | 0.9 | 0.9 | 0.9 | 0.9 | 0.9 | 0.9 |
| | | Dipropylene glycol | - | - | - | - | - | - |
| | | Ethanol | - | - | - | - | - | - |
| | | Decaglycerin monomyristic acid ester | 3.0 | 3.0 | 3.0 | 3.0 | 3.0 | 3.0 |
| | | Squalane | - | - | - | - | - | - |
| | | Glycerin | 35.0 | 35.0 | 35.0 | 35.0 | 35.0 | 35.0 |
| | Liquid B | Glycerin | - | - | - | - | - | - |
| | | Lecithin | 4.0 | 4.0 | 4.0 | 4.0 | 4.0 | 4.0 |
| | | Iodoproynyl butylcarbamate | 0.01 | 0.01 | 0.01 | 0.01 | 0.01 | 0.01 |
| | | Water | 56.5 | 56.5 | 56.5 | 56.5 | 56.5 | 56.5 |
| Temperature at the time of preparing crude dispersion liquid | | | 110°C | 110°C | 110°C | 110°C | 110°C | 110°C |
| Pressure (shearing force) at the time of emulsification | | | 245 MPa | 245 MPa | 245 MPa | 245 MPa | 245 MPa | 245 MPa |
| Evaluation | Particle size of ceramide dispersion particles | | 7 nm | 8 nm | 8 nm | 11 nm | 14 nm | 16 nm |
| | Skin care effect | | 5.0 | 4.6 | 4.6 | 2.4 | 2.4 | 2.4 |

[Table 2]

| | | | Example 7 | Example 8 | Example 9 | Example 10 | Example 11 | Example 12 | Example 13 | Example 14 | Example 15 | Example 16 |
|---|---|---|---|---|---|---|---|---|---|---|---|---|
| Formulation (part) | Liquid A | Ceramide I | 0.3 | 0.3 | 0.3 | 0.3 | 0.3 | 0.3 | 0.3 | 0.3 | 0.003 | 0.3 |
| | | Ceramide EOP | - | - | - | - | - | - | - | - | - | - |
| | | Ceramide EOS | - | - | - | - | - | - | - | - | - | - |
| | | Ceramide II | - | - | - | - | - | - | - | - | - | - |
| | | Ceramide III | - | - | - | - | - | - | - | - | - | - |
| | | Ceramide VI | - | - | - | - | - | - | - | - | - | - |
| | | Cholesterol | 0.3 | 0.3 | 0.3 | 0.3 | 0.3 | 0.3 | 0.3 | 0.3 | 0.3 | 0.3 |
| | | 1,3-butlene glycol | - | - | - | 0.9 | 0.9 | 0.9 | 0.9 | 0.9 | 0.9 | 0.9 |
| | | Dipropylene glycol | 0.9 | - | - | - | - | - | - | - | - | - |
| | | Ethanol | - | - | 0.9 | - | - | - | - | - | - | - |
| | | Decaglycerin monomyristic acid ester | 3.0 | 3.0 | 3.0 | 3.0 | 3.0 | 3.0 | 3.0 | 3.0 | 3.0 | - |
| | | Sucrose stearic acid ester | - | - | - | - | - | - | - | - | - | 3.0 |
| | | Squalane | - | - | - | - | 0.3 | 0.03 | - | - | - | - |
| | | Glycerin | 35.0 | 35.0 | 35.0 | 35.0 | 35.0 | 35.0 | 35.0 | 2.1 | 35.0 | 35.0 |
| | Liquid B | Glycerin | - | - | - | - | - | - | - | - | - | - |
| | | Lecithin | 4.0 | 4.0 | 4.0 | - | 4.0 | 4.0 | 4.0 | 4.0 | 4.0 | 4.0 |
| | | Iodopropynyl butylcarbamate | 0.01 | 0.01 | 0.01 | 0.01 | 0.01 | 0.01 | 0.01 | 0.01 | 0.01 | 0.01 |
| | | Water | 56.5 | 57.4 | 56.5 | 60.5 | 56.2 | 56.5 | 56.5 | 89.4 | 56.8 | 56.5 |
| Temperature at the time of preparing crude dispersion liquid | | | 110°C | 110°C | 110°C | 110°C | 110°C | 110°C | 110°C | 110°C | 110°C | 110°C |

(continued)

| | | Example 7 | Example 8 | Example 9 | Example 10 | Example 11 | Example 12 | Example 13 | Example 14 | Example 15 | Example 16 |
|---|---|---|---|---|---|---|---|---|---|---|---|
| Pressure (shearing force) at the time of emulsification | | 245 MPa | 245 MPa | 245 MPa | 245 MPa | 245 MPa | 245 MPa | 50 MPa | 245 MPa | 245 MPa | 245 MPa |
| Evaluation | Particle size of ceramide dispersion particles | 10 nm | 18 nm | 50 nm | 24 nm | 19 nm | 16 nm | 58 nm | 40 nm | 9 nm | 40 nm |
| | Skin care effect | 4.6 | 3.2 | 3.6 | 3.6 | 3.0 | 3.6 | 4.0 | 3.8 | 3.0 | 3.0 |

[Table 3]

| | | | Comparative example 1 | Comparative example 2 | Comparative example 3 | Comparative example 4 | Comparative example 5 | Comparative example 6 | Comparative example 7 | Comparative example 8 |
|---|---|---|---|---|---|---|---|---|---|---|
| Formulation (part) | Liquid A | Ceramide I | 0.3 | - | - | 0.3 | - | - | 0.3 | 0.003 |
| | | Ceramide EOP | - | - | - | - | - | - | - | - |
| | | Ceramide EOS | - | - | - | - | - | - | - | - |
| | | Ceramide II | - | 0.3 | - | - | 0.3 | - | - | - |
| | | Ceramide III | - | - | - | - | - | - | - | - |
| | | Ceramide VI | - | - | 0.3 | - | - | 0.3 | - | - |
| | | Cholesterol | 0.3 | 0.3 | 0.3 | 0.3 | 0.3 | 0.3 | 0.3 | 0.3 |
| | | 1,3-butylene glycol | 0.9 | 0.9 | 0.9 | 0.9 | 0.9 | 0.9 | 0.9 | 0.9 |
| | | Dipropylene glycol | - | - | - | - | - | - | - | - |
| | | Ethanol | - | - | - | - | - | - | - | - |
| | | Decaglycerin monomyristic acid ester | 3.0 | 3.0 | 3.0 | 3.0 | 3.0 | 3.0 | 3.0 | 3.0 |
| | | Squalane | - | - | - | - | - | - | - | - |
| | | Glycerin | - | - | - | 35.0 | 35.0 | 35.0 | - | 45.0 |
| | Liquid B | Glycerin | 35.0 | 35.0 | 35.0 | - | - | - | - | - |
| | | Lecithin | 4.0 | 4.0 | 4.0 | 4.0 | 4.0 | 4.0 | 4.0 | 4.0 |
| | | Iodopropynyl butylcarbamate | 0.01 | 0.01 | 0.01 | 0.01 | 0.01 | 0.01 | 0.01 | 0.01 |
| | | Water | 56.5 | 56.5 | 56.5 | 56.5 | 56.5 | 56.5 | 91.5 | 46.8 |
| Temperature at the time of preparing crude dispersion liquid | | | 110°C | 110°C | 110°C | 80°C | 80°C | 80°C | 110°C | 110°C |
| Pressure (shearing force) at the time of emulsification | | | 245 MPa | 245 MPa | 245 MPa | 245 MPa | 245 MPa | 245 MPa | 245 MPa | 245 MPa |

(continued)

| | | Comparative example 1 | Comparative example 2 | Comparative example 3 | Comparative example 4 | Comparative example 5 | Comparative example 6 | Comparative example 7 | Comparative example 8 |
|---|---|---|---|---|---|---|---|---|---|
| Evaluation | Particle size of ceramide dispersion particles | 57 nm | 80 nm | 77 nm | 170 nm | 158 nm | 212 nm | 70 nm | 9 nm |
| | Skin care effect | 1.6 | 1.2 | 1.2 | 1.4 | 1.0 | 1.0 | 1.6 | 1.8 |

In Tables 1, 2, and 3, "-" means that the corresponding component is not formulated. Furthermore, the components described in Tables 1, 2, and 3 are as follows.

- Ceramide I (trade name: CERAMIDE I, INCI name: Ceramide 1, Evonik Industries AG)
- Ceramide EOP (trade name: CERAMIDE EOP27, INCI name: Ceramide EOP, Evonik Industries AG)
- Ceramide EOS (trade name: CERAMIDE EOS27, INCI name: Ceramide EOS, Evonik Industries AG)
- Ceramide II (trade name: CERAMIDE TIC-001, INCI name: Ceramide 2, Takasago International Corporation)
- Ceramide III (trade name: CERAMIDE III, INCI name: Ceramide 3, Evonik Industries AG)
- Ceramide IV (trade name: CERAMIDE VI, INCI name: Ceramide 611, Evonik Industries AG)
- Cholesterol (trade name: CHOLESTEROL JSQI, Nippon Fine Chemical)
- 1,3-Butylene glycol (DAICEL CORPORATION, polyhydric alcohol)
- Dipropylene glycol (KOYO FINE CHEMICAL CORP., polyhydric alcohol)
- Decaglycerin monomyristic acid ester (trade name: NIKKOL DECAGLYN 1-M, HLB: 14.0, Nikko Chemicals Co., Ltd., nonionic surfactant)
- Sucrose stearic acid ester (trade name: RYOTO SUGAR ESTER S-1670, HLB: 16.0, Mitsubishi-Kagaku Foods Corporation, nonionic surfactant)
- Squalane (trade name: NIKKOL SQUALANE, Nikko Chemicals Co., Ltd.)
- Glycerin (concentrated glycerin for makeup, Kao Corporation., polyhydric alcohol)
- Lecithin (trade name: SLP-PC70, Tsuji Oil Mills co., Ltd.)
- Iodopropynyl butylcarbamate (trade name: GLYCACIL, LONZA Japan)

As is evident from Tables 1, 2, and 3, unexpectedly, all of the ceramide dispersion compositions of Examples 1 to 16 obtained by the manufacturing method of the present invention contained fine ceramide dispersion particles and had an excellent skin care effect.

The ceramide dispersion compositions of Examples 1 to 3 using the compound represented by Formula (I) had finder ceramide dispersion particles and a markedly better skin care effect, compared to the ceramide dispersion compositions of Examples 4 to 6 using ceramide other than the compound.

The ceramide dispersion compositions of Examples 1 and 7 using both of the glycerin and the diol compound (1,3-butylene glycol or dipropylen glycol) as a polyhydric alcohol had finer ceramide dispersion particles and better skin care effect, compared to Example 8 using either the glycerin or the diol compound.

The ceramide dispersion composition of Example 13 contained ceramide dispersion particles having a particle size equivalent to the particle size of the ceramide dispersion particles in the ceramide dispersion composition of Comparative example 1, but the skin care effect thereof was markedly better than that of the ceramide dispersion composition of Comparative example 1.

Therefore, according to the present invention, it is possible to provide a ceramide dispersion composition, which contains fine ceramide dispersion particles and exhibits an excellent skin care effect when being used in an external preparation for skin, by a simple method.

## Claims

**1.** A method for manufacturing a ceramide dispersion composition, comprising:

obtaining a crude dispersion liquid by performing a dispersion treatment in a state where a first mixture, which contains ceramide, a nonionic surfactant, and a polyhydric alcohol in an amount of 10 times to 12,000 times as much as the mass of the ceramide, is heated to a temperature of equal to or higher than 100°C; and
mixing the crude dispersion liquid with water or a water-containing composition to obtain a second mixture and subjecting the second mixture to a high-pressure emulsification treatment.

**2.** The method for manufacturing a ceramide dispersion composition according to claim 1,
wherein the polyhydric alcohol is at least one kind of polyhydric alcohol selected from the group consisting of glycerin and a diol compound.

**3.** The method for manufacturing a ceramide dispersion composition according to claim 2,
wherein the polyhydric alcohol is glycerin and at least one kind of diol compound.

**4.** The method for manufacturing a ceramide dispersion composition according to any one of claims 1 to 3,
wherein lecithin is used.

5. The method for manufacturing a ceramide dispersion composition according to any one of claims 1 to 4, wherein the nonionic surfactant is a polyglycerin fatty acid ester.

6. The method for manufacturing a ceramide dispersion composition according to any one of claims 1 to 5, wherein the ceramide dispersion composition does not contain an oleaginous component staying in a liquid form at 25°C or contains an oleaginous component staying in a liquid form at 25°C in an amount of equal to or less than 1% by mass.

7. The method for manufacturing a ceramide dispersion composition according to any one of claims 1 to 6, wherein the ceramide is a compound represented by the following Formula (I),

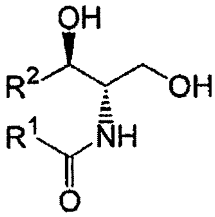

・・・(I)

in Formula (I), $R^1$ represents a linear or branched hydrocarbon group which has 40 to 55 carbon atoms in total, contains an ester bond in a carbon chain, and may contain one partial structure or two or more partial structures selected from the group consisting of a multiple bond, an aromatic ring, and an aromatic heterocyclic ring in the carbon chain; and $R^2$ represents an alkyl group having 11 to 21 carbon atoms that may have a hydroxyl group or an alkenyl group having 11 to 21 carbon atoms that may have a hydroxyl group.

8. The method for manufacturing a ceramide dispersion composition according to claim 1, wherein the ceramide dispersion composition containing ceramide dispersion particles having an average particle size of less than 60 nm.

9. The method for manufacturing a ceramide dispersion composition according to claim 1, wherein the content of the ceramide in the first mixture is from 0.00001% by mass to 10% by mass with respect to the total mass of the ceramide dispersion composition.

10. The method for manufacturing a ceramide dispersion composition according to claim 1, wherein the content of the nonionic surfactant in the first mixture is from 1 time to 70 times as much as the mass of the ceramide.

11. The method for manufacturing a ceramide dispersion composition according to claim 1, wherein the first mixture further comprises cholesterol.

12. The method for manufacturing a ceramide dispersion composition according to claim 1, wherein the high-pressure emulsification treatment is a dispersion treatment in which a shearing force of equal to or greater than 50 MPa is applied to the second mixture.

**Patentansprüche**

1. Verfahren zur Herstellung einer Ceramiddispersionszusammensetzung, umfassend:

   Erhalten einer Rohdispersionsflüssigkeit durch Ausführen einer Dispersionsbehandlung in einem Zustand, in dem eine erste Mischung, die Ceramid, ein nichtionisches Tensid und einen mehrwertigen Alkohol in einer Menge, 10 mal bis 12000 mal so hoch wie die Masse des Ceramids, enthält auf eine Temperatur von gleich oder höher als 100°C erhitzt wird; und
   Mischen der Rohdispersionsflüssigkeit mit Wasser oder einer Wasser-enthaltenden Zusammensetzung, um eine zweite Mischung zu erhalten und Unterwerfen der zweiten Mischung einer Hochdruckemulgierbehandlung.

2. Verfahren zur Herstellung einer Ceramiddispersionszusammensetzung nach Anspruch 1, wobei der mehrwertige Alkohol mindestens eine Sorte mehrwertiger Alkohol ist, die ausgewählt ist aus der Gruppe bestehend aus Glycerin und einer Diolverbindung.

3. Verfahren zur Herstellung einer Ceramiddispersionszusammensetzung nach Anspruch 2, wobei der mehrwertige Alkohol Glycerin und mindestens eine Sorte einer Diolverbindung ist.

4. Verfahren zur Herstellung einer Ceramiddispersionszusammensetzung nach einem der Ansprüche 1 bis 3, wobei Lecithin verwendet wird.

5. Verfahren zur Herstellung einer Ceramiddispersionszusammensetzung nach einem der Ansprüche 1 bis 4, wobei das nichtionische Tensid ein Polyglycerinfettsäureester ist.

6. Verfahren zur Herstellung einer Ceramiddispersionszusammensetzung nach einem der Ansprüche 1 bis 5, wobei die Ceramiddispersionszusammensetzung keine ölige Komponente enthält, die bei 25°C in flüssiger Form verbleibt oder eine ölige Komponente, die bei 25°C in flüssiger Form verbleibt in einer Menge gleich oder weniger als 1 Masse% enthält.

7. Verfahren zur Herstellung einer Ceramiddispersionszusammensetzung nach einem der Ansprüche 1 bis 6, wobei das Ceramid eine durch die folgende Formel (I) dargestellte Verbindung ist,

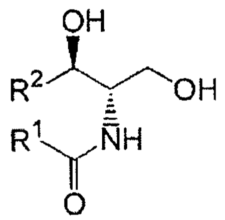 · · · (I)

wobei in Formel (I), $R^1$ eine lineare oder verzweigte Kohlenwasserstoffgruppe darstellt, die insgesamt 40 bis 55 Kohlenstoffatome hat, die eine Esterbindung in einer Kohlenstoffkette enthält und eine Teilstruktur oder zwei oder mehrere Teilstrukturen enthalten kann, ausgewählt aus der Gruppe bestehend aus einer Mehrfachbindung, einem aromatischen Ring und einem aromatischen heterozyklischen Ring in der Kohlenstoffkette; und $R^2$ eine Alkylgruppe mit 11 bis 21 Kohlenstoffatomen darstellt, die eine Hydroxylgruppe aufweisen kann oder eine Alkenylgruppe mit 11 bis 21 Kohlenstoffatomen, die eine Hydroxylgruppe aufweisen kann.

8. Verfahren zur Herstellung einer Ceramiddispersionszusammensetzung nach Anspruch 1, wobei die in der Ceramiddispersionszusammensetzung enthaltenen Ceramiddispersionspartikel eine durchschnittliche Partikelgröße von weniger als 60 nm aufweisen.

9. Verfahren zur Herstellung einer Ceramiddispersionszusammensetzung nach Anspruch 1, wobei der Gehalt an dem Ceramid in der ersten Mischung von 0,00001 Masse% bis 10 Masse%, bezogen auf die Gesamtmasse der Ceramiddispersionszusammensetzung beträgt.

10. Verfahren zur Herstellung einer Ceramiddispersionszusammensetzung nach Anspruch 1, wobei der Gehalt an nichtionischem Tensid in der ersten Mischung einmal bis 70 mal so hoch wie die Masse des Ceramids ist.

11. Verfahren zur Herstellung einer Ceramiddispersionszusammensetzung nach Anspruch 1, wobei die erste Mischung ferner Cholesterol enthält.

12. Verfahren zur Herstellung einer Ceramiddispersionszusammensetzung nach Anspruch 1, wobei die Hochdruckemulgierbehandlung eine Dispersionsbehandlung ist, in der eine Scherkraft von gleich oder größer als 50 MPa auf die zweite Mischung aufgebracht wird.

**Revendications**

1. Procédé destiné à fabriquer une composition de dispersion de céramide, comprenant les étapes consistant à :

obtenir un liquide de dispersion brut en réalisant un traitement de dispersion dans un état où un premier mélange, lequel contient un céramide, un agent de surface non ionique, et un alcool polyhydrique en une quantité égale à 10 fois à 12 000 fois la masse du céramide, est chauffé à une température supérieure ou égale à 100 °C, et

mélanger le liquide de dispersion brut avec de l'eau ou une composition contenant de l'eau pour obtenir un second mélange, et soumettre le second mélange à un traitement d'émulsification à haute pression.

2. Procédé destiné à fabriquer une composition de dispersion de céramide selon la revendication 1, dans lequel l'alcool polyhydrique est au moins un type d'alcool polyhydrique sélectionné parmi le groupe consistant en de la glycérine et un composé diol.

3. Procédé destiné à fabriquer une composition de dispersion de céramide selon la revendication 2, dans lequel l'alcool polyhydrique est de la glycérine et au moins un type de composé diol.

4. Procédé destiné à fabriquer une composition de dispersion de céramide selon l'une quelconque des revendications 1 à 3, dans lequel on utilise de la lécithine.

5. Procédé destiné à fabriquer une composition de dispersion de céramide selon l'une quelconque des revendications 1 à 4, dans lequel l'agent de surface non ionique est un ester d'acide gras de polyglycérine.

6. Procédé destiné à fabriquer une composition de dispersion de céramide selon l'une quelconque des revendications 1 à 5, dans lequel la composition de dispersion de céramide ne contient pas de composant oléagineux restant sous une forme liquide à 25 °C, ou contient un agent oléagineux restant sous une forme liquide à 25 °C dans une quantité inférieure ou égale à 1% en masse.

7. Procédé destiné à fabriquer une composition de dispersion de céramide selon l'une quelconque des revendications 1 à 6, dans lequel le céramide est un composé représenté par la formule suivante (I),

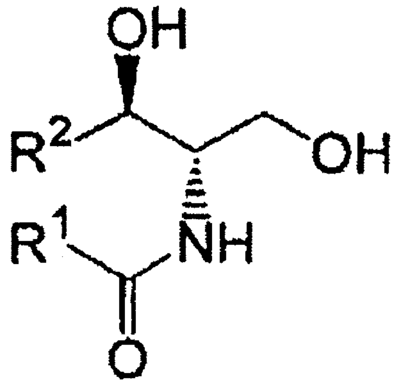

• • • (I)

dans la formule (I), $R^1$ représente un groupe hydrocarbure linéaire ou ramifié, lequel présente de 40 à 55 atomes de carbone au total, contient une liaison ester dans une chaîne carbone, et peut contenir une structure partielle ou deux ou plus structures partielles sélectionnées parmi le groupe consistant en une liaison multiple, un cycle aromatique, et un cycle hétérocylique aromatique dans la chaîne carbone, et $R^2$ représente un group alkyle présentant de 11 à 21 atomes de carbone, lequel peut présenter un groupe hydroxyle, ou un groupe alcényle présentant de 11 à 21 atomes de carbone, lequel peut présenter un groupe hydroxyle.

8. Procédé destiné à fabriquer une composition de dispersion de céramide selon la revendication 1, dans lequel la composition de dispersion de céramide contient des particules de dispersion céramide présentant une taille de particules moyenne inférieure à 60 nm.

9. Procédé destiné à fabriquer une composition de dispersion de céramide selon la revendication 1, dans lequel la teneur en céramide dans le premier mélange est comprise dans une plage allant de 0,00001 % en masse à 10 % par rapport à la masse totale de la composition de dispersion de céramide.

10. Procédé destiné à fabriquer une composition de dispersion de céramide selon la revendication 1, dans lequel la

teneur en agent de surface non ionique dans le premier mélange est égale à 1 fois à 70 fois la masse du céramide.

**11.** Procédé destiné à fabriquer une composition de dispersion de céramide selon la revendication 1, dans lequel le premier mélange comprend en outre du cholestérol.

**12.** Procédé destiné à fabriquer une composition de dispersion de céramide selon la revendication 1, dans lequel le traitement d'émulsification à haute pression est un traitement de dispersion où une force de cisaillement supérieure ou égale à 50 MPa est appliquée au second mélange.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2006335693 A
- JP 2013224314 A
- US 20060057091 A